# EUROPEAN PATENT SPECIFICATION

(11) **EP 0 987 029 B1**
(45) Date of publication and mention of the grant of the patent: **22.01.2003**
(21) Application number: 98402142.8
(22) Date of filing: 28.08.1998
(51) Int. Cl.: A61K 48/00, A61K 47/48, C08F 126/02

(54) **Use of a catonic polymer for the preparation of a complex with nucleic acid and related compositions**
Verwendung eines kationischen Polymer zur Herstellung von Nukleinsäure-Komplexe und verwandte Zusammensetzungen
Utilisation des polymères cationiques pour la préparation de complexes d' acides nucléiques et compositions apparentées

(43) Date of publication of application: 22.03.2000
(73) Proprietor: TRANSGENE S.A., 67082 Strasbourg Cédex (FR)
(72) Inventor: Kolbe, Hanno V.J., 67 204 Achenheim (FR); Boussif, Otmane, 67 000 Strassbourg (FR)
(74) Representative: VOSSIUS & PARTNER

(56) References cited:
- EP-A- 0 580 078
- WO-A-96/22792
- WO-A-97/29118
- KABANOV A V ET AL: "DNA COMPLEXES WITH POLYCATIONS FOR THE DELIVERY OF GENETIC MATERIALINTO CELLS" BIOCONJUGATE CHEMISTRY, vol. 6, no. 1, 1 January 1995, pages 7-20, XP000494803
- BEHR J -P ET AL: "EFFICIENT GENE TRANSFER INTO MAMMALIAN PRIMARY ENDOCRINE CELLS WITHLIPOPOLYAMINE-COATED DNA" PROCEEDINGS OF THE NATIONAL ACADEMY OF SCIENCES OF USA, vol. 86, September 1989, pages 6982-6986, XP002057565
- FELGNER P L ET AL: "LIPOFECTON: A HIGHLY EFFICIENT, LIPID-MEDIATED DNA-TRANSFECTION PROCEDURE" PROCEEDINGS OF THE NATIONAL ACADEMY OF SCIENCES OF USA, vol. 84, no. 21, November 1987, pages 7413-7417, XP000602252
- GAO X ET AL: "A NOVEL CATIONIC LIPOSOME REAGENT FOR EFFICIENT TRANSFECTION OF MAMMALIAN CELLS" BIOCHEMICAL AND BIOPHYSICAL RESEARCH COMMUNICATIONS, vol. 179, no. 1, 30 August 1991, pages 280-285, XP000572654

## Description

The present invention concerns complexes and compositions comprising cationic polymers and nucleic acids. More particularly, this invention relates to a method for transferring nucleic acids into a cell, more particularly a mammalian cell.

Gene therapy has generally been conceived as principally applicable to heritable deficiency diseases (cystic fibrosis, dystrophies, haemophilias,...) where permanent cure may be effected by introducing a functional gene. However, a much larger group of diseases, notably acquired diseases (cancer, AIDS, multiple sclerosis,...) might be treatable by transiently engineering host cells to produce beneficial proteins.

Another application of gene therapy is vaccination. In this regard, the immunogenic product encoded by the nucleic acid introduced into cells of a vertebrate may be expressed and secreted or be presented by said cells in the context of the major histocompatibility antigens, thereby eliciting an immune response against the expressed immunogen. Functional nucleic acid can be introduced into cells by a variety of techniques resulting in either transient expression of the gene of interest, referred to as transient transfection, or permanent transformation of the host cells resulting from incorporation of the nucleic acid into the host genome.

Successful gene therapy depends on the efficient delivery to and expression of genetic information within the cells of a living organism. Most delivery mechanisms used to date involve viral vectors, especially adeno- and retroviral vectors. Viruses have developed diverse and highly sophisticated mechanisms to achieve this goal including crossing of the cellular membrane, escape from endosomes and lysosomal degradation, and finally delivery of their genome to the nucleus followed by expression of the viral genome. In consequence, viruses have been used in many gene delivery applications in vaccination or gene therapy applied to humans. The use of viruses suffers from a number of disadvantages: retroviral vectors can not accommodate large-sized DNA (e.g. the dystrophin gene, around 13 kb), the retroviral genome is integrated into host cell DNA and may thus cause genetic changes in the recipient cell and infectious viral particles could disseminate within the organism or into the environment; adenoviral vectors can induce a strong immune response in treated patients (Mc Coy et al, 1995, Human Gene Therapy, 6, 1553-1560; Yang et al., 1996, Immunity, 1, 433-442). Nevertheless, despite these drawbacks, viral vectors are currently the most useful delivery systems because of their efficiency.

In 1990, Wolff et al. (Science, 247, 1465-1468) have shown that injection of naked RNA or DNA, without any special delivery system, directly into mouse skeletal muscle results in expression of reporter genes within the muscle cells. Nevertheless, although these results indicate that nucleic acid by itself is capable of end-up in certain cells *in vivo,* the efficiency of the transfection actually observed remains very limited due, in particular, to the polyanionic nature of nucleic acids which limits their passage through negatively-charged cell membranes.

Various methods have been proposed in the literature based on the use of non-viral synthetic vectors to improve intracellular uptake of nucleic acids which present potential advantages with respect to large-scale production, safety, targeting of transfectable cells, low immunogenicity, and the capacity to deliver large fragments of DNA. Thus, in 1989, Felgner et al. (Nature, 337, 387-388) proposed the use of cationic lipids in order to facilitate the introduction of large anionic molecules such as nucleic acids into cells. These cationic lipids are capable of forming complexes with anionic molecules, thus tending to neutralize the negative charges of these molecules allowing to compact the complex, and favoring its introduction into the cell. Example of lipid-mediated transfection compounds are DOTMA (Felgner et al., 1987, PNAS, 84, 7413-7417), DOGS or Transfectam™ (Behr et al.,1989, PNAS, 86, 6982-6986), DMRIE or DORIE (Felgner et al., 1993, Methods 5, 67-75), DC-CHOL (Gao et Huang, 1991, BBRC, 179, 280-285), DOTAP™ (McLachlan et al., 1995, Gene Therapy, 2,674-622) or Lipofectamine™.

Other non-viral delivery systems have been developed which are based on polymer-mediated transfection. There have been many reports on the use for cellular delivery of anionic polymers such as, for example, polyamidoamine (Haensler et Szoka, 1993, Bioconjugate Chem., 4, 372-379), dendritic polymer (WO 95/24221), polyethylene imine or polypropylene imine (WO 96/02655), polylysine (US-A-5,595,897 or FR-A-2 719 316).

However, several studies (for example, Mahato et al., J. Pharm. Sci., 1995, 84, 1267-1271, Thierry et al., 1995, PNAS 92, 9742-9746) have shown that the transfer efficiency of the complexed nucleic acids into the cells, especially in the case of *in vivo* transfer, can vary in function of the interaction between the complexes and the cell membranes, the cell type involved, the lipid composition of the cationic components, the size of the complexes formed with the anionic molecules and, especially, the ratio of the positive to negative charges of the different components of the complex. Currently, very little is known concerning the mechanisms which enable the interaction of the complexes with the cell membranes and the transfer of the complexes into the cell, and the ongoing research remains highly empirical. Consequently, there exists a need to develop improved methods for increasing the intracellular uptake of nucleic acids based on complexes having properties different from those already described.

Polyvinylamine has already been used in pharmaceutical compositions as a cholestorolemia regulator within specific diets (EP-A-580 078, EP-A-580 079) but does not incitate a skilled person to use it in gene therapy application.

US-A-5,629,184 describes compositions for cellular uptake of polynucleotides comprising copolymers of vinyl alcohol and varying levels of vinylamine (from 0.5 to 75 mole %). These authors have tested their compositions *in vitro* and have concluded that in general, high levels of amino lead to cytotoxicity resulting in disintegration of cells while lower levels lead to an endosomal/lysosomal distribution of the polynucleotides.

French patent applications FR 97/09209 and FR 97/15807 (filing numbers) show that polyallylamine of general formula : 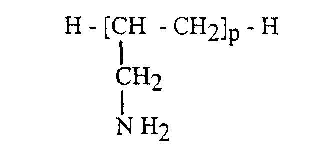 with p ranging from 2 to 1000
are relatively toxic for cells and therefore are not indicated for gene therapy applications.

The applicant has now identified new complexes and compositions comprising cationic polymers which surprisingly provide an efficient system to achieve cellular delivery of nucleic acid into cells with low toxicity and which can therefore find an application in *in vivo* gene therapy.

Thus, the present invention first concerns a complex comprising :
a) at least one polymer of the general formula : 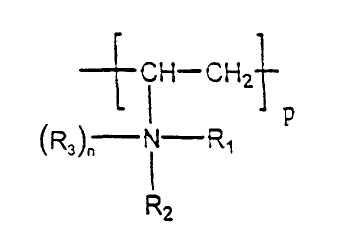 wherein :
   the degree of polymerization p ranges from 2 to 1000000, preferably from 10 to 10000, more preferably from 50 to 2000,
   R₁, R₂ and R₃, independently of one another in each [CH - CH₂] repeat, are selected from H, alkyl of 1 to 20 carbon atoms or aryl of 5 to 7 carbon atoms,
   n is 0 or 1, with the proviso that at least one n is 1 in the full length of the polymer and
b) at least one nucleic acid.

According to the present invention, said alkyl or aryl group can optionally be repeated, can be linear or branched and can optionally be substituted. Furthermore, R₁, R₂ and/or R₃ can be linked in order to cyclize the molecule.

According to a preferred embodiment, the degree of polymerization p is selected in order to obtain polymers having a molecular mass which ranges between 1 and 1000 kDa, preferably between 7 and 450 kDa.

Said polymers can be synthetized for example according to the method described in Lewis, E.A., Barker, J., St. Pierre, T. (1981) "Calorometric Titration of Poly(vinylamine) and Poly(iminoethylene)." Macromolecules, 14, 546-551.

Furthermore, due to the presence of amino functions and R₁, R₂ and/or R₃ group, the polymer of said complex can be substituted, directly or via a spacer such as heterobifunctional reactives such as SPDP or SCMC, functionalized PEG which are well known by a skilled man (Mattson et al 1993, Mol. Biol. Reports, 17, 167-183). The substituents can be at least one element of those widely disclosed in scientific publications: labelling molecules (for example, see molecules disclosed in US-A-4,711,955) enabling, for example, visualization of the distribution of the polymer, or of complexed nucleic acid, after *in vitro* or *in vivo* administration; cell targeting molecules (ligands) or anchoring molecules ; elements facilitating penetration into the cell, lysis of endosomes or even intracellular transport towards the nucleus. These elements may be composed of all or part of sugars, glycol, peptides (e.g. GRP, Gastrin Releasing Peptide), oligonucleotides, lipids (especially those with C2-C22, hormones, vitamins, antigens, antibodies (or fragments thereof), specific membrane receptor ligands, ligands capable of reaction with an anti-ligand, fusogenic peptides, nuclear localization peptides, or a combination of said compounds, e.g. galactosyl residues to target the asialoglycoprotein receptor on the surface of hepatocytes, the INF-7 fusogenic peptide derivated from the HA-2 subunit of the influenza virus hemagglutinin (Plank et al. 1994, J. Biol. Chem. 269, 12918-12924) for membrane fusion, or a nuclear signal sequence derived from the T-antigen of the SV40 virus (Lanford and Butel, 1984, Cell *37*, 801-813) or from the EBNA-1 protein of the Epstein Barr virus (Ambinder et al., 1991, J. Virol. *65*, 1466-1478). Furthermore, the amino groups and R₁, R₂ and/or R₃ group of the polymer according to the invention can be totally or partially substituted with hydrophilic groups R as those described in French patent applications FR 97/09209 and FR 97/15807, the content of which is herewith incorporated as part of the present application.

Such substituted polymers can be obtained easily using the techniques described in the literature, especially by chemical coupling, notably by using protector groups such as trifluoroacetyl, Fmoc (9-fluorenylmethoxycarbonyl) or BOC (tert-butyl oxycarbonyl) on the amine moiety. Selective removal of a protector group then allows coupling of the targeting element, and then complete deprotection of the polymer (Greene T.W. and Wuts P.G.M., 1991, Protective groups in organic synthesis. Ed. J. Wiley & Sons, Inc. New York).

In order to form complexes with nucleic acids, polymers according to the invention are in cationic form. This means either a) they are in a protonated form by binding a proton to at least one nitrogen atom present on the amino group, or b) they are in ammonium form by binding with e.g. -CH₃, -C₂H₅ or -CH₂CH₂OH to at least one nitrogen atom present on the amino group or c) they are in forms a) and b). In these cases, said cationic polymers would be associated with one or several biologically acceptable anions, such as, for example, trifluoroacetate, monoethylsulfate, acetate, iodide, chloride, bromide, etc.

The nucleic acid comprised in said complex may be a DNA and/or RNA fragment, single or double-stranded, linear or circular, natural or synthetic, modified or not (see US-A-5,525,711, US-A-4,711,955 or EP-A-302 175 for modification examples) defining a fragment or a portion of a nucleic acid, without size limitation. It may be, *inter alia*, a genomic DNA, a cDNA, a mRNA, an antisense RNA, a ribosomal RNA, a ribozyme, a tRNA or DNA encoding such RNAs. "Polynucleotides" and "nucleic acids" are synonyms with regard to the present invention. The nucleic acid may also be in the form of a plasmid or linear polynucleotide which contains at least one coding sequence of nucleic acid that can be transcribed and translated to generate polypeptide, ribozyme, antisense or other molecule of interest upon delivery to a cell. The nucleic acid can also be an oligonucleotide which is to be delivered to the cell, e.g., for antisense or ribozyme functions. According to the invention, said nucleic acid can also be co-formulated with viral proteins, or cationic lipids, or cationic polymers.

In a preferred embodiment, both DNA and RNA can be delivered to cells to form therein a polypeptide of interest which may stay within the cell or which will be secreted from the cell. In a more preferred embodiment, plasmid DNA is preferred. If the nucleic acids contain the proper genetic information, they will direct the synthesis of relatively large amounts of the encoded polypeptide. When the nucleic acids delivered to the cells encode an immunizing polypeptide, the use according to the invention can be applied to achieve improved and effective immunity against infectious agents, including intracellular viruses, and also against tumor cells. The genetic information necessary for expression by a target cell comprises all the elements required for transcription of said DNA into mRNA and for translation of mRNA into polypeptide. Transcriptional promoters suitable for use in various vertebrate systems are well known. For example, suitable promoters include viral promoters RSV, MPSV, SV40, CMV or 7.5k vaccinia promoter, inducible promoters, etc. Nucleic acids can also include intron sequences, targeting sequences, transport sequences, sequences involved in replication or integration. Said sequences have been reported in the literature and can be readily obtained by those skilled in the art. Nucleic acids can also be stabilized with specific components such as spermine.

According to the invention, the nucleic acid can be homologous or heterologous to the expressing target cells. Advantageously said nucleic acid encodes all or part of a polypeptide, especially a therapeutic or prophylactic polypeptide. A polypeptide is understood to be any translational product of a polynucleotide regardless of size, and whether glycosylated or not, and includes peptides and proteins. Therapeutic polypeptides include as a primary example those polypeptides that can compensate for defective or deficient proteins in an animal, or those that act through toxic effects to limit or remove harmful cells from the body. They can also be immunity-conferring polypeptides which act as endogenous immunogens to provoke a humoral or cellular response, or both. Examples of encoded polypeptides according to the invention are enzyme, hormone, cytokine, membrane receptor, structural polypeptide, transport polypeptide, adhesine, ligand, transcription factor, transduction factor, replication factor, stabilisation factor, antibody, more especially CFTR, dystrophin, factors VIII or IX, E6 or E7 from HPV, MUC1, BRCA1, interferon β, interferon γ, interleukin IL-2, IL-4, IL-6, IL-7, IL-12, GM-CSF (Granulocyte Macrophage Colony Stimulating Factor), tk gene from Herpes Simplex type 1 virus (HSV-1), p53, VEGF. The polynucleotide can also code for an antibody. In this regard, antibody encompasses whole immunoglobulin of any class, chimeric antibodies and hybrid antibodies with dual or multiple antigen or epitope specificities, and fragments, such as F(ab)₂, Fab', Fab including hybrid fragments and anti-idiotypes (US-A-4,699,880).

In a specific embodiment of the invention, the size of cationic polymer /nucleic acid complexes according to the invention is of small size (less than 5 µm, more advantageously less than 500 nm and preferably ranges between 20 and 200 nm). Complex size may be selected for optimal use in a particular application.

Measurements of the complex size can be achieved by a number of techniques including, but not limited to, dynamic laser light scattering (photon correlation spectrocopy, PCS), as well as other techniques known to those skilled in the art (see, Washington, Particle Size Analysis in Pharmaceutics and other Industries, Ellis Horwood, New York, 1992, 135-169).

The invention is also directed to methods for preparing said complexes which are characterized in that one or more said polymer is mixed with one or more nucleic acid, and that said complex is then recovered.

Concentration of the negatively-charged nucleic acid, which may be added to the cationic polymer to form said complexes of the invention ranges from 10 µg to 5000 µg per ml pharmaceutical composition. In a preferred embodiment of the invention, the concentration of nucleic acid ranges from 100 µg/ml to 1000 µg/ml.

According to a particular embodiment, the complex further comprises at least one adjuvant likely to improve formation of said complex between said cationic polymer and said nucleic acid, or to facilitate transport of said complex across the cell membrane and prevent endosomal degradation.

Therefore, the invention also relates to a complex wherein said adjuvant is selected from the group consisting in positively or negatively-charged, neutral or zwitterionic lipids, for example a triglyceride, a diglyceride, cholesterol (see for example US-A-5,438,044), and, in particular a positively or negatively-charged, neutral or zwitterionic lipid which is or derivates from a phosphatidylethanolamine (PE), phosphatidylcholine, phosphocholine, dioleoylphosphatidylethanolamine (DOPE), sphingomyelin, ceramide or cerebroside, cationic lipid such as for example glycerolipids (see PCT/FR 98/00250) or PEGylated lipids (see WO 98/08489) or lysomotropic agents such as chloroquine.

The ratio of polymer to said adjuvant (on a weight to weight basis) can range from 1:0.1 to 1:10, but this ratio may be adapted according to the type of compound. In a preferred embodiment said weight ratio is 1:1. A skilled person is capable of handling these minor adjustments. It is also possible to use a mixture of positively or negatively-charged, neutral and/or zwitterionic lipids.

The complexes of the invention, also named polyplexes, may also be characterized by their theoretical charge ratio (+/-), which is the ratio of the positive charges provided by the cationic polymer to the negative charges provided by the nucleic acid in the complex, assuming that all potentially cationic groups are in fact in the cationic state and all potentially anionic groups are in fact in the anionic state. In general, an excess of positive charges on the complex facilitates binding of the complex to the negatively-charged cell surface. To obtain such a ratio, the calculation shall take into account all negative charges in the nucleic acid and shall then adjust the quantity of cationic polymer necessary to obtain the desired theoretical charge ratio indicated above. The quantities and the concentrations of the other ingredients shall be adjusted in function of their respective molar masses and their number of positive charges. The ratio is not specifically limited: quantities are selected so that the ratio between the number of positive charges in the cationic polymer and the number of negative charges in the nucleic acid is between 0.05 and 20, notably between 2.5 and 15, and preferably around 2.5 to 10.

The invention also encompasses a pharmaceutical composition comprising at least one complex as previously disclosed. Said pharmaceutical compositions are particularly useful for the delivery of polynucleotides to cells or tissues of a subject in the scope of a gene therapeutic method but are not limited to such use. The term "gene therapy method" is preferably understood as a method for the introduction of a polynucleotide into cells either in vivo or by introduction into cells *in vitro* followed by re-implantation into a subject. "Gene therapy" in particular concerns the case where the gene product is expressed in a target tissue as well as the case where the gene product is excreted, especially into the blood stream. According to the present invention, nucleic acid, polynucleotide, oligonucleotides or gene are synonyms.

Preferably, the pharmaceutical composition furthermore comprises a pharmaceutically acceptable carrier. The carrier is preferably isotonic, hypotonic or weakly hypertonic and has a relatively low ionic strength, such as provided by a sucrose solution. Furthermore, it may contain any relevant solvents, aqueous or partly aqueous liquid carriers comprising sterile, pyrogen-free water, dispersion media, coatings, and equivalents. The pH of the pharmaceutical preparation is suitably adjusted and buffered.

The pharmaceutical composition in accordance with the present invention can be administered into a vertebrate tissue. This administration may be made by intradermal, subdermal, intravenous, intramuscular, intranasal, intracerebral, intratracheal, intraarterial, intraperitoneal, intravesical, intrapleural, intracoronary or intratumoral injection, by means of a syringe or other devices. Transdermal administration is also contemplated, as are inhalation or aerosol routes.

According to the present invention, the pharmaceutical composition can be administered into target tissues of the vertebrate body including those of muscle, skin, brain, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor, etc.

Applied to *in vivo* gene therapy, this invention allows repeated administration to the patient without any risk of the administered preparation to induce a significant immune reaction. Administration may be by single or repeated dose, once or several times after a certain period of time. Repeated administration allows a reduction in the dose of active substance, in particular DNA, administered at a single time. The route of administration and the appropriate dose vary in function of several parameters, for example the individual patient, the illness being treated, or the nucleic acid being transferred.

The invention more particularly pertains to a pharmaceutical composition comprising at least one of the complexes described above and also incorporating at least one adjuvant capable of improving the transfection capacity of said complex. Adjuvants may be selected in the group consisting in a chloroquine, protic polar compounds such as propylene glycol, polyethylene glycol, glycerol, EtOH, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives.

According to the invention, "cells" include prokaryote cells and eukaryote cells, yeast cells, plant cells, human or animal cells, in particular mammalian cells. In particular, cancer cells should be mentioned. The invention can be applied *in vivo* to the interstitial or luminal space of tissues in the lungs, the trachea, the skin, the muscles, the brain, the liver, the heart, the spleen, the bone marrow, the thymus, the bladder, the lymphatic system, the blood, the pancreas, the stomach, the kidneys, the ovaries, the testicles, the rectum, the peripheral or central nervous system, the eyes, the lymphoid organs, the cartilage, the endothelium. In preferred embodiments, the cell will be a muscle cell, a hematopoietic system stem cell or an airways cell, more especially a tracheal or pulmonary cell, and preferably a cell of the respiratory epithelium.

The present invention also encompasses a process for transferring a nucleic acid into cells wherein said process comprises contacting said cells with at least one complex or composition according to the invention. This process may be applied by direct administration of said complex or composition to cells of the animal *in vivo,* or by *in vitro* treatment of cells which were recovered from the animal and then re-introduced into the animal body (*ex vivo* process). In *in vitro* application, cells cultivated on an appropriate medium are placed in contact with a suspension consisting of complexes or composition of the invention. After an incubation time, the cells are washed and recovered. Introduction of the active substance can be verified (eventually after lysis of the cells) by any appropriate method.

The introduction or transfer process is by itself well known. "Introduction or transfer" means that the negatively-charged active substance is transferred into the cell and is located, at the end of the process, inside said cell or within or on its membranes. If the active substance is a nucleic acid, this is called "transfection". Transfection can be verified by any appropriate method, for example by measuring the expression of said gene or by measuring the concentration of the expressed protein or its mRNA, or by measuring its biological effect.

In the case of *in vivo* treatment according to the invention, in order to improve the transfection rate, the patient may undergo a macrophage depletion treatment prior to administration of the pharmaceutical preparations described above. Such a technique is described in the literature (refer particularly to Van Rooijen et al., 1997, TibTech, 15, 178-184).

The invention more particularly concerns the use of polymers of the general formula : 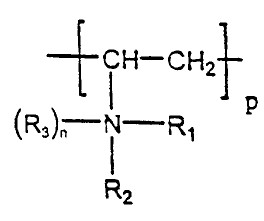 wherein:
the degree of polymerization p ranges from 2 to 10E6, preferably from 10 to 10E4, more preferably from 50 to 2000,
R₁, R₂ and R₃, independently of one another in each [CH - CH₂] repeat, are selected from H, alkyl of 1 to 20 carbon atoms or aryl of 5 to 7 carbon atoms, n is 0 or 1 with the proviso that at least one n is 1 in the full length of the polymer or of a complex between said polymer and nucleic acid for the preparation of a pharmaceutical composition for gene therapy use and more specifically for use in method for intracellular delivery of nucleic acids.

According to a preferred embodiment, the degree of polymerization p is selected in order to obtain polymers having a molecular weight which ranges between 1 and 1000 kDa, preferably between 7 and 450 kDa.

Moreover, according to its cationic property, said polymer can also be used for the preparation of pharmaceutical compositions for therapy wherein intracellular delivery of anionic molecule is needed. Such anionic molecules should not be restrictively understood as nucleic acid and can also consist of proteins, drugs etc.

Lastly, the invention relates to a cell transfected with a complex such as that described above, in particular a prokaryote cell, a yeast or eukaryote cell, notably a mammalian cell, and more especially a cancerous cell. The invention is particularly effective for airways cells, especially tracheal or pulmonary cells, and even more so for cells of the respiratory epithelium.

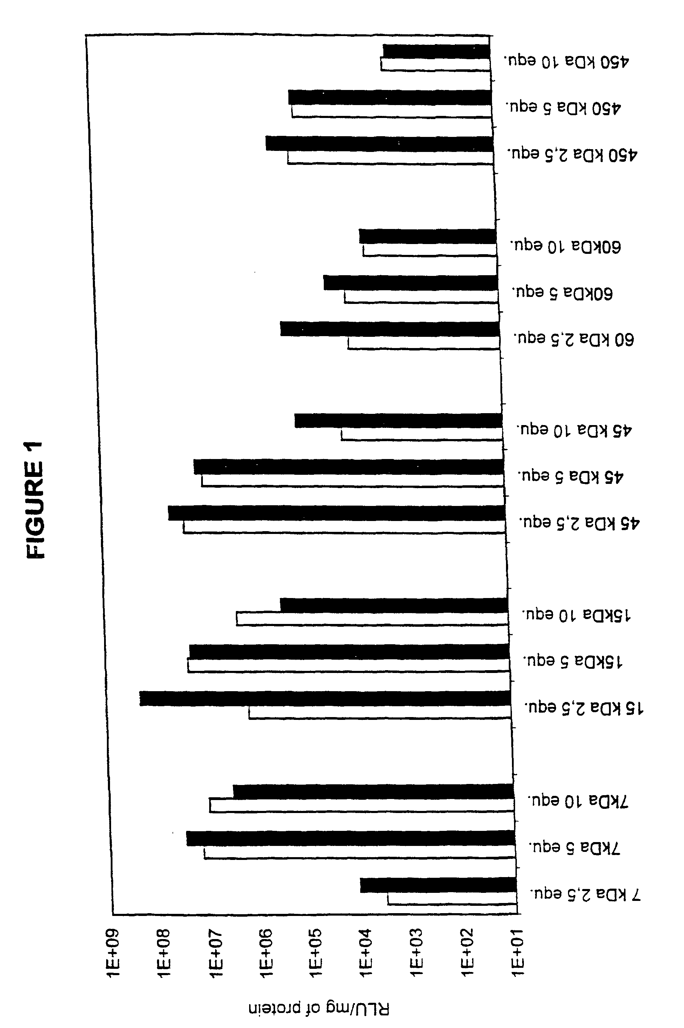

**Figure 1**: A549 cells (human lung carcinoma cells) were transfected with 2 µg pTG11033 and the desired amount of the desired polymer to reach the desired N/P equivalents (2.5, 5 and 10 equ., respectively; see legend). The expression was stopped 48 hours later. Black bars represent results of transfection in the presence of chloroquine (100 µM).

### EXAMPLES

### Cells

A549 cells (pulmonary carcinoma cell line) are maintenained in culture in DMEM medium supplemented with 10% fetal calf serum, 286 mg/ml glutamine and 2 g/l glucose, in an incubator at 37°C and 5% CO₂. 24 hours prior to transfection cells are seeded in 48 multi-well plate to reach 60-80% confluence at transfection-time.

### Plasmid

pTG11033 (FR 97/09209 and FR 97/15807) plasmid encoding the *Photinus pyralis* luciferase gene under the control of the cytomegalovirus enhancer/promoter and containing the intron HMG1 is used.

### Solutions

The amino polymer solutions were prepared by dissolving weighed amounts of the corresponding hydrochloride salts in distillated water. The concentrations are expressed as monomer poly(amine hydrochloride); we have considered the polymer as totally protonated; MM of 79 Da/monomer).

### Preparation of polymer/DNA polyplexes and protocol of transfection

The polymer/DNA complexes were obtained as followed: 2 µg of plasmid and the desired amount of cationic polymer [from 10 mM (in term of protonated amine) stock solutions, in water at pH 7.4; 1 N/P equivalent corresponds to the amount of polymer necessary to have one amino group (N) per phosphate group (P) of nucleic acid (330 Da mean MM)] were each diluted in 50 µl 5% glucose solution and vortexed. The two solutions were then mixed and gently vortexed. After 30 min the complexes were added to the cells. Before adding the transfection complexes, the medium (DMEM, GIBCO BRL) was removed and replaced by 0.5 ml of serum-free medium. Immediately thereafter, the plate was centrifuged for 5 min at 1500 rpm (approx. 280 g). After 3 h, the medium was replaced by 0.5 mL of medium supplemented with 10% fetal calf serum. When transfection was performed in the presence of 100 µM chloroquine, 5 µl of 10 mM chloroquine solution was added to the medium just before adding polyplexes. After 48 h, the medium was removed, 100 µl lysis buffer (Promega) were added and cells were frozen at -80 °C until analysis for luciferase activity. 20 µl of the supernatant were analysed using the luciferase assay system (Promega) in 96 multi-well plates (Biolumat LB 9500, Berthold, Wilbach, Germany). Each transfection was done in triplicate. Values are given as mean relative light units (RLU) per mg of cell protein (BCA assay, Pierce).
Like the majority of synthetic vectors the transfection efficiency depends on the polymer/DNA ratio. Optimal expression was always obtained with negative/positive charge ratio between 2.5 and 5. No significant toxicity was seen at all ratios tested. We also have shown that the transfection in the presence of 100 µM chloroquine could allow higher transgene expression.

## Claims

1. A complex comprising :
a) at least one polymer of the general formula : 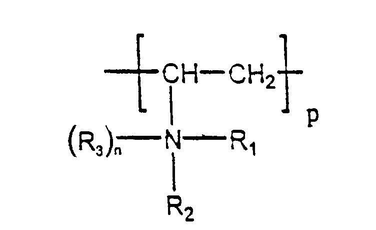 wherein:
the degree of polymerization p ranges from 2 to 1000000,
R₁, R₂ and R₃, independently of one another in each [CH - CH₂] repeat, are selected from H, alkyl of I to 20 carbon atoms or aryl of 5 to 7 carbon atoms,
n is 0 or 1, with the proviso that at least one n is 1 in the full length of the polymer, and
b) at least one nucleic acid.

2. The complex of claim 1, wherein p ranges from 10 to 10000.

3. The complex of claim 2, wherein R₁, R₂ and R₃ are H.

4. The complex of claims 1 to 3, wherein said polymer also contains one or more targeting elements bonded to the nitrogen atom or any of R₁, R₂ and R₃ of said polymer.

5. The complex of claim 4, wherein said targeting element is selected from the group consisting in all or part of sugars, glycol derivatives, peptides, oligonucleotides, lipids, hormones, vitamins, antigens, antibodies, specific membrane receptor ligands, ligands capable of reaction with an anti-ligand, fusogen peptides, nuclear localization peptides, or a combination of said compounds.

6. The complex of claims 1 to 5, wherein said nucleic acid is selected from the group consisting of cDNA, genome DNA, plasmid DNA, messenger RNA, antisense RNA, ribozymes, transfer RNA, ribosomal RNA, or DNA encoding for such types of RNA.

7. Complex of claim 6, wherein said nucleic acid includes a therapeutically useful gene sequence and the elements enabling its expression.

8. Complex of claims 1 to 6, wherein said complex-size is less than 5 µm.

9. Complex of claims 1 to 8, wherein the ratio between the number of positive charges in said polymer and the number of negative charges in said nucleic acid is between 0.05 and 20.

10. Complex of claims 1 to 9 further comprising at least one adjuvant selected from positively or negatively charged, neutral and zwitterionic lipids.

11. Complex of claim 10, wherein said adjuvant is selected from phosphatidylethanolamine (PE), phosphatidylcholine, phosphocholine, dioleylphosphatidylethanolamine (DOPE), sphingomyelin, ceramide or cerebroside or one of their derivates.

12. Complex of claim 10, wherein said adjuvant is a cationic lipid compound.

13. Complex of claims 10 to 12, wherein the ratio of polymer to said adjuvant ranges from 1:0.1 to 1:10 (w/w).

14. Pharmaceutical composition comprising at least one complex of claims 1-13.

15. Pharmaceutical composition of claim 14, wherein the concentration of the negatively-charged nucleic acid ranges from 10 µg to 5000 µg per ml composition.

16. Pharmaceutical composition of claim 15, wherein said concentration ranges from 100 µg to 1000 µg per ml composition.

17. Pharmaceutical composition of claim 16, further comprising at least one adjuvant selected in the group consisting in chloroquine, protic polar compounds such as propylene glycol, polyethylene glycol, glycerol, ethanol, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives.

18. Process for transferring a nucleic acid into cells wherein said process comprises contacting said cells with at least one complex of claims 1 to 13 or composition of claims 14 to 17.

19. Use of a polymer of the general formula : 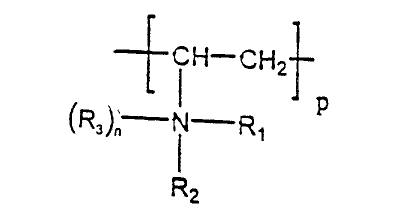 wherein:
the degree of polymerization p ranges from 2 to 1000000,
R₁, R₂ and R₃ are, independently of one another in each [CH - CH₂] repeat, selected from H or an alkyl of 1 to 20 carbon atoms or an aryl of 5 to 7 carbon atoms,
n is 0 or 1, with the proviso that at least one n is 1 in the full length of the polymer, for the preparation of a pharmaceutical composition for intracellular delivery of anionic molecules.

20. Use of a complex as defined in any one of claims 1 to 13 for the preparation of a pharmaceutical composition for intracellular delivery of nucleic acids.

21. Use according to claim 19 or 20, wherein said pharmaceutical composition is for administration by intramuscular injection, by inhalation, by intratracheal injection, by instillation, by aerosolization or by topical application.

22. Use of a complex as defined in claims 1 to 13, or a composition as defined in claims 14 to 17 for transferring *in vitro* of at least one nucleic acid into a cell.

23. Use of claim 22, wherein said cell is a mammalian cell.

24. Cell transfected by a complex as defined in claims 1 to 13.

25. A process for the preparation of a complex as defined in any of claims 1 to 13, comprising the step of mixing at least one said polymer with at least one nucleic acid and recovering the complex.

## Patentansprüche

1. Komplex, umfassend:
a) mindestens ein Polymer der allgemeinen Formel: 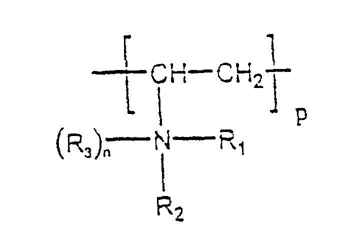 wobei:
der Polymerisationsgrad p von 2 bis 1000000 beträgt,
R₁, R₂ und R₃ unabhängig voneinander in jeder [CH-CH₂]-Wiederholungseinheit aus H, einem Alkylrest mit 1 bis 20 Kohlenstoffatomen oder einem Arylrest mit 5 bis 7 Kohlenstoffatomen ausgewählt sind,
n 0 oder 1 ist, mit der Maßgabe, dass mindestens ein n in der gesamten Länge des Polymers 1 ist, und
b) mindestens einer Nukleinsäure.

2. Komplex gemäß Anspruch 1, wobei p 10 bis 10000 beträgt.

3. Komplex gemäß Anspruch 2, wobei R₁, R₂ und R₃ H sind.

4. Komplex gemäß den Ansprüchen 1 bis 3, wobei das Polymer auch eines oder mehrere Targetelemente enthält, welche an das Stickstoffatom oder an einen der Reste R₁, R₂ und R₃ des Polymers gebunden sind.

5. Komplex gemäß Anspruch 4, wobei das Targetelement ganz oder teilweise aus Zuckern, Glycolderivaten, Peptiden, Oligonucleotiden, Lipiden, Hormonen, Vitaminen, Antigenen, Antikörpern, spezifischen Membranrezeptorliganden, Liganden, welche zur Reaktion mit einem Antiliganden befähigt sind, fusogenen Peptiden, Kernlokalisationspeptiden oder einer Kombination dieser Verbindungen ausgewählt ist.

6. Komplex gemäß den Ansprüchen 1 bis 5, wobei die Nukleinsäure aus cDNS, Genom-DNS, Plasmid-DNS, Messenger-RNS, Antisens-RNS, Ribozymen, Transfer-RNS, ribosomaler RNS oder DNS, welche derartige RNS-Arten kodiert, ausgewählt ist.

7. Komplex gemäß Anspruch 6, wobei die Nukleinsäure eine therapeutisch nützliche Gensequenz enthält und die Elemente deren Expression ermöglicht.

8. Komplex gemäß den Ansprüchen 1 bis 6, wobei die Komplexgröße weniger als 5 µm beträgt.

9. Komplex gemäß den Ansprüchen 1 bis 8, wobei das Verhältnis zwischen der Zahl an positiven Ladungen in dem Polymer und der Zahl der negativen Ladungen in der Nukleinsäure zwischen 0,05 und 20 beträgt.

10. Komplex gemäß den Ansprüchen 1 bis 9, ferner mindestens einen Hilfsstoff umfassend, welcher aus positiv oder negativ geladenen, neutralen und zwitterionischen Lipiden ausgewählt ist.

11. Komplex gemäß Anspruch 10, wobei der Hilfsstoff aus Phosphatidylethanolamin (PE), Phosphatidylcholin, Phosphocholin, Dioleylphosphatidylethanolamin (DOPE), Spingomyelin, Ceramid oder Cerebrosid oder einem ihrer Derivate ausgewählt ist.

12. Komplex gemäß Anspruch 10, wobei der Hilfsstoff eine kationische Lipidverbindung ist.

13. Komplex gemäß den Ansprüchen 10 bis 12, wobei das Verhältnis des Polymers zu dem Hilfsstoff 1 : 0,1 bis 1 : 10 (w/w) beträgt.

14. Arzneimittel, umfassend mindestens einen Komplex gemäß den Ansprüchen 1 bis 13.

15. Arzneimittel gemäß Anspruch 14, wobei die Konzentration der negativ geladenen Nukleinsäure 10 µg bis 5000 µg pro ml der Zusammensetzung beträgt.

16. Arzneimittel gemäß Anspruch 15, wobei die Konzentration 100 µg bis 1000 µg pro ml der Zusammensetzung beträgt.

17. Arzneimittel gemäß Anspruch 16, ferner mindestens einen Hilfsstoff umfassend, welcher aus Chloroquin, protischen, polaren Verbindungen, wie Propylenglycol, Polyethylenglycol, Glycerin, Ethanol, 1-Methyl-L-2-pyrrolidon oder deren Derivaten, oder aprotischen, polaren Verbindungen, wie Dimethylsulfoxid (DMSO), Diethylsulfoxid, Di-n-propylsulfoxid, Dimethylsulfon, Sulfolan, Dimethylformamid, Dimethylacetamid, Tetramethylharnstoff, Acetonitril oder deren Derivaten ausgewählt ist.

18. Verfahren zum Übertragen einer Nukleinsäure in Zellen, wobei das Verfahren das Zusammenbringen der Zellen mit mindestens einem Komplex gemäß den Ansprüchen 1 bis 13 oder einer Zusammensetzung gemäß den Ansprüchen 14 bis 17 umfasst.

19. Verwendung eines Polymers der allgemeinen Formel: 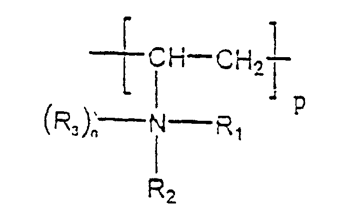 wobei:
der Polymerisationsgrad p von 2 bis 1000000 beträgt,
R₁, R₂ und R₃ unabhängig voneinander in jeder [CH-CH₂]-Wiederholungseinheit aus H, einem Alkylrest mit 1 bis 20 Kohlenstoffatomen oder einem Arylrest mit 5 bis 7 Kohlenstoffatomen ausgewählt sind,
n 0 oder 1 ist, mit der Maßgabe, dass mindestens ein n in der gesamten Länge des Polymers 1 ist, zur Herstellung eines Arzneimittels zur intrazellularen Abgabe von anionischen Molekülen.

20. Verwendung eines Komplexes gemäß einem der Ansprüche 1 bis 13 zur Herstellung eines Arzneimittels zur intrazellularen Abgabe von Nukleinsären.

21. Verwendung gemäß Anspruch 19 oder 20, wobei das Arzneimittel zur Verabreichung durch intramuskuläre Injektion, durch Inhalation, durch intratracheale Injektion, durch Einflößen, durch Aerosolierung oder durch topische Anwendung bestimmt ist.

22. Verwendung eines Komplexes gemäß den Ansprüchen 1 bis 13 oder einer Zusammensetzung gemäß den Ansprüchen 14 bis 17 zur *in vitro* Übertragung mindestens einer Nukleinsäure in eine Zelle.

23. Verwendung gemäß Anspruch 22, wobei die Zelle eine Säugerzelle ist.

24. Zelle, welche durch einen Komplex gemäß den Ansprüchen 1 bis 13 transfiziert wurde.

25. Verfahren zur Herstellung eines Komplexes gemäß einem der Ansprüche 1 bis 13, umfassend Mischen mindestens eines der Polymere mit mindestens einer Nukleinsäure und Isolieren des Komplexes.

## Revendications

1. Complexe comprenant:
a) au moins un polymère de formule générale: 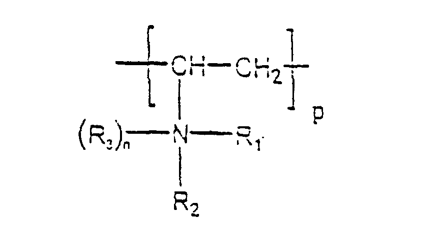 dans laquelle:
le degré de polymérisation p est compris entre 2 et 1,000,000,
dans chaque motif [CH - CH₂], R₁, R₂, et R₃ sont sélectionnés indépendamment l'un de l'autre parmi H, un alkyle comptant de 1 à 20 atomes de carbone ou un aryle comptant de 5 à 7 atomes de carbone, n représente 0 ou 1, avec la condition qu'au moins un n représente 1 sur toute la longueur du polymère, et
b) au moins un acide nucléique.

2. Complexe selon la revendication 1, dans lequel p est compris entre 10 et 10,000.

3. Complexe selon la revendication 2, dans lequel R₁, R₂ et R₃ représentent H.

4. Complexe selon les revendications 1 à 3, dans lequel le dit polymère contient également un ou plusieurs éléments de ciblage liés à l'atome d'azote, ou à l'un quelconque parmi R₁, R₂ et R₃ du dit polymère.

5. Complexe selon la revendication 4, dans lequel le dit élément de ciblage est sélectionné dans le groupe constitué en tout ou en partie de sucres, de dérivés de glycol, de peptides, d'oligonucléotides, de lipides, d'hormones, de vitamines, d'antigènes, d'anticorps, de ligands spécifiques à des récepteurs de membrane, de ligands capables de réagir avec un antiligand, de peptides fusogènes, de peptides de localisation nucléaire ou d'une combinaison des dits composés.

6. Complexe selon les revendications 1 à 5, dans lequel le dit acide nucléique est choisi dans le groupe constitué de l'ADNc, de l'ADN de génome, de l'ADN de plasmide, de l'ARN messager, de l'ARN antisens, des ribozymes, de l'ARN de transfert, de l'ARN de ribosome ou de l'ADN codant ces types d'ARN.

7. Complexe selon la revendication 6, dans lequel le dit acide nucléique comprend une séquence de génique thérapeutiquement utile, et les éléments permettant son expression.

8. Complexe selon les revendications 1 à 6, dans lequel la taille du dit complexe est inférieure à 5 µm.

9. Complexe selon les revendications 1 à 8, dans lequel le rapport entre le nombre de charges positives dans le dit polymère, et le nombre de charges négatives dans le dit acide nucléique est compris entre 0,05 et 20.

10. Complexe selon les revendications 1 à 9, comprenant en outre au moins un adjuvant sélectionné parmi des lipides chargés positivement ou chargés négativement, neutres et zwitterioniques.

11. Complexe selon la revendication 10, dans lequel le dit adjuvant est sélectionné parmi la phosphatidyléthanolamine (PE), la phosphatidylcholine, la phosphocholine, la dioléylphophatidyléthanolamine (DOPE), la sphingomyéline, le céramide ou le cérébroside ou l'un de leurs dérivés.

12. Complexe selon la revendication 10, dans lequel le dit adjuvant est un composé de lipide cationique.

13. Complexe selon les revendications 10 à 12, dans lequel le rapport entre les polymères et le dit adjuvant est compris entre 1:0,1 et 1:10 (poids/poids).

14. Composition pharmaceutique comprenant au moins un complexe selon les revendications 1 à 13.

15. Composition pharmaceutique selon la revendication 14, dans laquelle la concentration de l'acide nucléique chargé négativement est comprise entre 10 µg et 5000 µg par ml de composition.

16. Composition pharmaceutique selon la revendication 15, dans laquelle la dite concentration est comprise entre 100 µg et 1000 µg par ml de composition.

17. Composition pharmaceutique selon la revendication 16, comprenant en outre au moins un adjuvant sélectionné dans le groupe constitué de la chloroquine, de composés polaires protiques tels que le propylène glycol, le polyéthylène glycol, le glycérol, l'éthanol, la 1-méthyl-L-2-pyrrolidone ou leurs dérivés, ou de composés polaires aprotiques tels que le sulfoxyde de diméthyle (DMSO), le sulfoxyde de diéthyle, le sulfoxyde de di-n-propyle, la diméthylsulfone, le sulfolane, le formamide de diméthyle, l'acétamide de diméthyle, la tétraméthylurée, l'acétonitrile ou leurs dérivés.

18. Procédé pour transférer un acide nucléique dans des cellules, le dit procédé comprenant la mise en contact des dites cellules avec au moins un complexe selon les revendications 1 à 13, ou une composition selon les revendications 14 à 17.

19. Utilisation d'un polymère de formule générale: 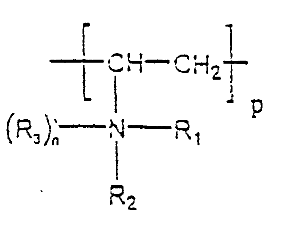 dans laquelle:
le degré de polymérisation p est compris entre 2 et 1,000,000,
dans chaque motif [CH - CH₂], R₁, R₂ et R₃ sont sélectionnés indépendamment l'un de l'autre parmi H ou un alkyle comptant de 1 à 20 atomes de carbone ou un aryle comptant de 5 à 7 atomes de carbone,
n représente 0 ou 1, avec la condition qu'au moins un n représente 1 sur toute la longueur du polymère, pour la préparation d'une composition pharmaceutique en vue de la délivrance intracellulaire de molécules anioniques.

20. Utilisation d'un complexe selon l'une quelconque des revendications 1 à 13, pour la préparation d'une composition pharmaceutique pour délivrance intracellulaire d'acides nucléiques.

21. Utilisation selon la revendication 19 ou 20, dans laquelle la dite composition pharmaceutique est destinée à être administrée par injection intramusculaire, par inhalation, par injection intratrachéale, par instillation, par application en aérosol ou par application topique.

22. Utilisation d'un complexe selon les revendications 1 à 13 ou d'une composition selon les revendications 14 à 17, pour le transfert *in vitro* d'au moins un acide nucléique dans une cellule.

23. Utilisation selon la revendication 22 dans laquelle la dite cellule est une cellule de mammifère.

24. Cellule transfectée par un complexe selon les revendications 1 à 13.

25. Procédé pour la préparation d'un complexe selon l'une quelconque des revendications 1 à 13, comprenant les étapes consistant à mélanger au moins l'un des dits polymères avec au moins un acide nucléique, et à récupérer le complexe.
